# EUROPEAN PATENT APPLICATION

(11) **EP 1 192 920 A1**
(43) Date of publication of application: **03.04.2002**
(21) Application number: 00870220.1
(22) Date of filing: 29.09.2000
(51) Int. Cl.: A61F 11/10, A61L 15/42

(54) **Comfortable foam-based ear plugs**

(71) Applicant: The Procter & Gamble Company, Cincinnati, Ohio 45202 (US)
(72) Inventor: Graydon, Andrew Russell, Newcastel upon Tyne NE7 7RW (GB); Stoddart, Barry, Low Fell, Gateshead NE9 5AP (GB); Blyth, Kevin Graham, Whitley Bay, Tyne & Wear NE26 1QT (GB); Brunner, Gordon Francis, Cincinnati, Ohio 45243 (US)
(74) Representative: Canonici, Jean-Jacques

(57) **Abstract**

Disclosed are ear plug devices which are constructed of an open-celled foam material, preferably a polymeric foam prepared from a High Internal Phase Emulsion (HIPE). Such foams have certain cell size, resistance to compression and resiliency characteristics along with certain specified dimensions. Such devices can be comfortably inserted into and worn within the ear canal of a wearer and provide effective blocking or attenuation of bothersome or troublesome noise or sounds entering the human ear.

## Description

### Technical Field/Field of the Invention

This invention relates to ear plug devices which are constructed of foam material and which are useful for blocking or attenuating bothersome or troublesome noise entering the human ear.

### Background of the Invention

Ear plugs have long been used as devices which can cover the ear or are positioned within the ear canal and which serve to block or attenuate troublesome, bothersome or dangerous noise. Such ear plugs can be used, for example, to block or attenuate especially load noises encountered on the job, e.g., loud aircraft noise or loud industrial machinery. Ear plugs can also be used to attenuate normal noises or sounds which the ear plug users simply wishes to block for purposes of facilitating rest, relaxation or sleep.

Currently known ear plugs are made out of materials such as polyurethane foam, or wax or elastomeric plastic. In order to provide good sound blocking, they must have (a) the ability to form a good seal within the ear and (b) be made of a sound absorbing material. Unfortunately, in order to get a good seal, the plug exerts pressure against the ear canal. This pressure can lead to discomfort for the wearer who consequently may be disinclined to use the plug. Often, attempts to use an alternative material which places less pressure results in an unacceptable compromise in the fundamental sound absorbing properties of the material.

One approach for addressing this problem is to manufacture an ear plug which is custom-made to the ear shape of the wearer. However, due to the infinite variety in ear shapes and sizes, this approach is not compatible with cheap mass-production.

Another alternative is to make ear plugs in a range of sizes. This suffers the disadvantage of manufacturing complexity. Moreover, research has shown that consumers are frequently unable to select the correct size of ear plug for optimum performance.

Most designs are in-the-canal devices. (See, for example, the foam ear plugs disclosed in U.S. Patents 3,811,437; 4,434,794; 4,774,938; 5,203,352; 5,979,451; 5,792,998; 5,799,658; and 6,006,857.) Because the ear canal size and shape can vary widely, the most frequent approach in the art is to make the plug of a compressible and deformable material in a shape that is larger than most canals. The plug is then inserted into the ear in a highly compressed form and allowed to expand in order to form the crucial seal with the ear. However, this means that the plug, once fitted, remains in a compressed state and so exerts a pressure on the ear canal, which can be sensitive to pressure. It has been found, in fact, that comfort is compromised when the plug exerts a pressure of about 9 kPa or more on the walls of the ear canal.

Plugs are also made which are to be located at the entrance to the ear canal (EAR Shrooms). However, these devices can be difficult to keep in place and are not suitable for sleeping in.

Given the foregoing, there is a continuing need to identify and provide ear plug devices which are especially effective for attenuating undesired noise and sounds but which are acceptably comfortable to wear. Such ear plugs devices should be mass producible and easy to fit into a secure position within the ear canal of the ear plug user.

### Summary of the Invention

The present invention is directed to an ear plug, generally used in pairs, which is suitable for effectively attenuating loud or bothersome noises when positioned in the ear (or preferably in both ears) of a wearer. Such an ear plug comprises from 0.02 to 1.0 gram of a three-dimensional mass of an open-celled, compressible foam material. The foam material used to form this mass is open-celled foam having interconnected cells which range an average size from 10 to 250 microns. Preferably such a foam is prepared, for example by polymerization, from a High Internal Phase Emulsion (HIPE).

This open-celled foam of selected cell size is formed into a mass of material which has dimensions in its compressed state such that the mass is suitable for insertion into the ear canal of the wearer. The foam will also have a resistance to compression such that the mass formed therefrom exerts a static stress of from 1 to 15 kPa when the foam mass is compressed to 50% of its relaxed volume. Finally, the foam material used must be sufficiently resilient such that the foam mass returns to at least 90% of its original relaxed volume within from 2 to 60 seconds after it is released from being compressed to at least 80% of its relaxed volume.

The mass of foam material is useful as an ear plug herein can be formed by molding foam material into shapes of suitable size and configuration. Alternatively, foam materials in the form of strips or discs can be rolled or folded into suitable masses having the requisite shape and size for use as ear plugs. Ear plugs fashioned from foam materials of the type specified herein have an especially desirable balance of sound attenuation performance and comfort for the wearer.

### Brief Description of the Drawings

Figure 1 of the drawings illustrates an ear plug device of the present invention which has been molded into a generally funnel-shaped configuration.
Figure 2 (a and b) of the drawings illustrates the preparation of an ear plug device in conical form which has been made by folding a flat disc of foam material.

### Detailed Description of the Invention

The ear plugs of this invention are fashioned from a foam material, generally polymeric, having certain structural characteristics which make such materials especially useful in the ear plug context. Such foams are those which are generally characterized as "open-celled" and which have a certain selected size of the cells or pores which form the foam.

Generally the foam materials used for the ear plugs herein will be polymeric. Polymeric foams can in general be characterized as the structures which result when a relatively monomer-free gas or relatively monomer-free liquid is dispersed as bubbles in a polymerizable monomer-containing liquid, followed by polymerization of the polymerizable monomers in the monomer-containing liquid which surrounds the bubbles. The resulting polymerized dispersion can be in the form of a porous, solidified structure which is an aggregate of cells, the boundaries or walls of which cells comprise solid polymerized material. The cells themselves contain the relatively monomer-free gas or relatively monomer-free liquid which, prior to polymerization, had formed the "bubbles" in the liquid dispersion.

As described more fully hereafter, the preferred polymeric foam materials useful for fashioning the ear plugs of the present invention are those prepared by polymerizing a particular type of water-in-oil emulsion. Such an emulsion is formed from a relatively small amount of a polymerizable monomer-containing oil phase and a relatively larger amount of a relatively monomer-free water phase. This type of polymerizable emulsion in general is known in the art as a high internal phase emulsion or "HIPE".

In the HIPE reaction mixture, the relatively monomer-free, discontinuous "internal" water phase forms the dispersed "bubbles" surrounded by the continuous polymerizable monomer-containing oil phase. Subsequent polymerization of the monomers in the continuous oil phase forms the cellular foam structure.

For preferred HIPE emulsions herein which are polymerized to form polymeric foams, the oil phase of the HIPE comprises a monomer component. In the case of HIPE foams suitable for use as ear plugs, this monomer component is typically formulated to form a resulting polymer or copolymer having a glass transition temperature (Tg) of 35°C or lower, and typically from -10°C to 30°C. The method for determining Tg by Dynamic Mechanical Analysis (DMA) is described in the TEST METHODS section of U.S. Patent 5,650,222, Issued to Thomas A. DesMarais, et al. on July 22, 1997.

For HIPE foams useful as ear plugs, the monomer component comprises one or more monomers that tend to impart rubber-like properties to the resulting polymeric foam structure. Such monomers can produce high molecular weight (greater than 10,000) atactic amorphous polymers having Tgs of 25°C or lower. Monomers of this type include, for example, monoenes such as the (C₄ -C₁₄) alkyl acrylates such as butyl acrylate, hexyl acrylate, octyl acrylate, 2-ethylhexyl acrylate, nonyl acrylate, decyl acrylate, dodecyl (lauryl) acrylate, isodecyl acrylate tetradecyl acrylate, aryl acrylates and alkaryl acrylates such as benzyl acrylate, nonylphenyl acrylate, the (C₆ -C₁₆) alkyl methacrylates such as hexyl acrylate, octyl methacrylate, nonyl methacrylate, decyl methacrylate, isodecyl methacrylate, dodecyl (lauryl) methacrylate, tetradecyl methacrylate, (C₄ -C₁₂) alkyl styrenes such as p-n-octylstyrene, acrylamides such as N-octadecyl acrylamide, and polyenes such as 2-methyl-1,3-butadiene (isoprene), butadiene, 1,3-pentadiene (piperylene), 1,3-hexadiene, 1,3-heptadiene, 1,3-octadiene, 1,3-nonadiene, 1,3-decadiene, 1,3-undecadiene, 1,3-dodecadiene, 2-methyl-1,3-hexadiene, 6-methyl-1,3-heptadiene, 7-methyl-1,3-octadiene, 1,3,7-octatriene, 1,3,9-decatriene, 1,3,6-octatriene, 2,3-dimethyl-1,3-butadiene, 2-methyl-3-ethyl-1,3-butadiene, 2-methyl-3-propyl-1,3-butadiene, 2-amyl-1,3-butadiene, 2-methyl-1,3-pentadiene, 2,3-dimethyl-1 ,3-pentadiene, 2-methyl-3-ethyl-1,3-pentadiene, 2-methyl-3-propyl-1,3-pentadiene, 2,6-diethyl-1,3,7-octatriene, 2,7-dimethyl-1,3,7-octatriene, 2,6-dimethyl-1 ,3,6-octatriene, 2,7-dimethyl-1,3,6-octatriene, 7-methyl-3-methylene-1,6-octadiene (myrcene), 2,6-dimethyl-1,5,7-octatriene (ocimene), 1-methyl-2-vinyl-4,6-hepta-dieny-3,8-nonadienoate, 5-methyl-1,3,6-heptatriene, 2-ethylbutadiene, and mixtures of these monomers. Of these monomers, isodecyl acrylate, n-dodecyl acrylate and 2-ethylhexyl acrylate are the most preferred. The monomer will generally comprise 30% to 85%, more preferably from 50% to 70%, by weight of the oil phase monomer component.

For HIPE foams useful as ear plugs, the oil phase monomer component also typically comprises one or more co-monomers which are typically included to modify the Tg properties of the resulting polymeric foam structure, its modulus (strength), and its toughness. These monofunctional comonomer types can include styrene-based comonomers (e.g., styrene and ethyl styrene) or other monomer types such as methyl methacrylate where the related homopolymer is well known as exemplifying toughness. Another example of a monomer which confers a high level of toughness to the resulting HIPE foam is isoprene and related dienes such as piperylene and dimethylbutadiene. Of these comonomers, styrene, ethyl styrene, and mixtures thereof are particularly preferred for imparting toughness to the resulting polymeric foam structure. These comonomers can comprise up to 40% of the oil phase monomer component and will normally comprise from 5% to 40%, preferably from 10% to 35%, most preferably from 15% to 30%, by weight of the monomer component.

For HIPE foams useful as ear plugs, the oil phase monomer component also includes one or more polyfunctional crosslinking agents. The inclusion of these crosslinking agents tends to increase the Tg of the resulting polymeric foam as well as its strength, somewhat at the expense of its flexibility and resilience. Suitable crosslinking agents include any of those that can be employed in crosslinking rubbery diene monomers, such as divinylbenzenes, divinyltoluenes, divinylxylenes, divinylnaphthalenes divinylalkylbenzenes, divinylphenanthrenes, trivinylbenzenes, divinylbiphenyls, divinyldiphenylmethanes, divinylbenzyls, divinylphenylethers, divinyldiphenylsulfides, divinylfurans, divinylsulfone, divinylsulfide, divinyldimethylsilane, 1,1'-divinylferrocene, 2-vinylbutadiene, maleate, di-, tri-, tetra-, penta- or higher (meth)acrylates and di-, tri-, tetra-, penta- or higher (meth)acrylamides, including ethylene glycol dimethacrylate, neopentyl glycol dimethacrylate, 1,3-butanediol dimethacrylate, 1,4-butanediol dimethacrylate, 1,6-hexanediol dimethacrylate, 2-butenediol dimethacrylate, diethylene glycol dimethacrylate, hydroquinone dimethacrylate, catechol dimethacrylate, resorcinol dimethacrylate, triethylene glycol dimethacrylate, polyethylene glycol dimethacrylate; trimethylolpropane trimethacrylate, pentaerythritol tetramethacrylate, 1,3-butanediol diacrylate, 1,4-butanediol diacrylate, 1,6-hexanediol diacrylate, diethylene glycol diacrylate, hydroquinone diacrylate, catechol diacrylate, resorcinol diacrylate, triethylene glycol diacrylate, polyethylene glycol diacrylate; pentaerythritol tetraacrylate, 2-butenediol diacrylate, tetramethylene diacrylate, trimethyol propane triacrylate, pentaerythritol tetraacrylate, N-methylolacrylamide, 1,2-ethylene bisacrylamide, 1,4-butane bisacrylamide, and mixtures thereof.

The preferred polyfunctional crosslinking agents include divinylbenzene, ethylene glycol dimethacrylate, diethylene glycol dimethacrylate, 1,6-hexanediol dimethacrylate, 2-butenediol dimethacrylate, ethylene glycol diacrylate, diethylene glycol diacrylate, 1,6-hexanediol diacrylate, 2-butenediol diacrylate, trimethylolpropane triacrylate and trimethacrylate, and mixtures thereof. Divinyl benzene is typically available as a mixture with ethyl styrene in proportions of 55:45. These proportions can be modified so as to enrich the oil phase with one or the other component. It can be advantageous to enrich the mixture with the ethyl styrene component while simultaneously omitting inclusion of styrene from the monomer blend. The preferred ratio of divinyl benzene to ethyl styrene is from 30:70 to 55:45, most preferably from 35:65 to 45:55. The inclusion of higher levels of ethyl styrene imparts desirable toughness without increasing the Tg of the resulting copolymer to the degree that styrene does. The cross-linking agent can generally be included in the oil phase of the HIPE in an amount of from 3% to 40%, more preferably from 4% to 40%, most preferably from 5% to 40%, by weight of the monomer/co-monomer/crosslinker component (100% basis).

The major portion of the oil phase of these preferred HIPEs will comprise these monomers, co-monomers and crosslinking agents. It is essential that these monomers, co-monomers and crosslinking agents be substantially water-insoluble so that they are primarily soluble in the oil phase and not the water phase. Use of such substantially water-insoluble monomers ensures that HIPE of appropriate characteristics and stability will be realized.

It is, of course, highly preferred that the monomers, co-monomers and crosslinking agents used herein be of the type such that the resulting polymeric foam is suitably non-toxic and appropriately chemically stable. These monomers, co-monomers and cross-linking agents should preferably have little or no toxicity if present at very low residual concentrations during post-polymerization foam processing and/or use.

Of all of the foregoing possible reactive components of the oil phase of the preferred HIPE emulsions herein, the most preferred will comprise a mixture of monomers, co-monomers and crosslinkers of the following composition:

| | |
|---|---|
| Ethyl Hexyl Acrylate | 47%-70% of the reactive components |
| Styrene | 22%-40% of the reactive components |
| Di-Vinyl Benzene | 8%-20% of the reactive components |

Another component of the oil phase is an emulsifier (or emulsifier combination) which permits the formation of stable HIPE emulsions. Suitable emulsifiers for use herein can include any of a number of conventional emulsifiers applicable for use in low and mid-internal-phase emulsions. Usually, these emulsifiers are nonionic materials and can have a wide range of HLB values. Examples of some typical emulsifiers include sorbitan esters such as sorbitan laurates (e.g., SPAN® 20), sorbitan palmitates (e.g., SPAN® 40), sorbitan stearates (e.g., SPAN® 60 and SPAN® 65), sorbitan monooleates (e.g., SPAN® 80), sorbitan trioleates (e.g., SPAN® 85), sorbitan sesquioleates (e.g., EMSORB® 2502), and sorbitan isostearates (e.g., CRILL® 6); polyglycerol esters and ethers (e.g., TRIODAN® 20); polyoxyethylene fatty acids, esters and ethers such as polyoxyethylene (2) oleyl ethers, polyethoxylated oleyl alcohols (e.g. BRIJ® 92 and SIMUSOL® 92), etc.; mono-, di-, and triphosphoric esters such as mono-, di-, and triphosphoric esters of oleic acid (e.g., HOSTAPHAT), polyoxyethylene sorbitol esters such as polyoxyethylene sorbitol hexastearates (e.g., ATLAS® G-1050), ethylene glycol fatty acid esters, glycerol mono-isostearates (e.g., IMWITOR 780K), ethers of glycerol and fatty alcohols (e.g., CREMOPHOR WO/A), esters of polyalcohols, synthetic primary alcohol ethylene oxide condensates (e.g., SYNPERONIC A2), mono and diglycerides of fatty acids (e.g., ATMOS® 300), and the like.

Other preferred emulsifiers include the diglycerol esters derived from monooleate, monomyristate, monopalmitate, and monoisostearate acids. A preferred coemulsifier is ditallowdimethyl ammonium methyl sulfate. Mixtures of these emulsifiers are also particularly useful, as are purified versions of each, specifically sorbitan esters containing minimal levels of isosorbide and polyol impurities.

Preferred emulsifiers also include sorbitan monolaurate (e.g., SPAN® 20, preferably greater than 40%, more preferably greater than 50%, most preferably greater than 70% sorbitan monolaurate), sorbitan monooleate (e.g., SPAN® 80, preferably greater than 40%, more preferably greater than 50%, most preferably greater than 70% sorbitan monooleate), diglycerol monooleate (e.g., preferably greater than 40%, more preferably greater than 50%, most preferably greater than 70% diglycerol monooleate), diglycerol monoisostearate (e.g., preferably greater than 40%, more preferably greater than 50%, most preferably greater than 70% diglycerol monoisostearate), diglycerol monomyristate (e.g., preferably greater than 40%, more preferably greater than 50%, most preferably greater than 70% sorbitan monomyristate), the cocoyl (e.g., lauryl and myristoyl) ethers of diglycerol, and mixtures thereof.

The oil phase used to form the HIPE can comprise varying ratios of oily materials and emulsifier. The particular ratios selected will depend on a number of factors including the oily materials involved and the emulsifier used. Generally, the oil phase can comprise from 50 to 98% by weight oily materials and from 2% to 50% by weight emulsifier. Typically, the oil phase will comprise from 70% to 97% by weight of the oily materials and from 3% to 30% by weight emulsifier, and more typically from 85% to 97% by weight of the oily materials and from 3% to 15% by weight emulsifier.

For preferred HIPEs used to make polymeric foams for ear plugs, the oil phase will generally comprise from 70% to 98% by weight monomer/co-monomer/crosslinker component and from 2% to 30% by weight emulsifier component. Preferably, the oil phase will comprise from 80% to 97% by weight monomer/co-monomer/crosslinker component and from 3% to 20% by weight emulsifier component. More preferably, the oil phase will comprise from 90% to 97% by weight monomer/comonomer/crosslinker component and from 3% to 10% by weight emulsifier component.

In addition to the monomer/co-monomer/crosslinker and emulsifier components, the oil phase of these preferred HIPEs can contain other optional components. One such optional component is an oil soluble polymerization initiator of the general type well known to those skilled in the art, such as described in US Patent 5,290,820 (Bass et al.), Issued Mar. 1, 1994. Another possible optional component is a substantially water insoluble solvent for the monomer and emulsifier components. Use of such a solvent is not preferred, but if employed will generally comprise no more than 10% by weight of the oil phase.

A preferred optional oil phase component is an antioxidant such as a Hindered Amine Light Stabilizer (HALS), such as bis-1,2,2,5,5-pentamethylpiperidinyl) sebacate (Tinuvin 765) or a Hindered Phenolic Stabilizer (HPS) such as Irganox 1076 and t-butylhydroxyquinone. Another preferred optional component is a plasticizer such as dioctyl azelate, dioctyl sebacate or dioctyl adipate. Other optional components include fillers, colorants, fluorescent agents, opacifying agents, chain transfer agents, and the like.

The internal water phase of the HIPE is generally an aqueous solution containing one or more dissolved components. One dissolved component of the water phase can be a water-soluble electrolyte. The dissolved electrolyte minimizes the tendency of the components in the oil phase to also dissolve in the water phase. For preferred HIPEs used to make polymeric foams for ear plugs, this is believed to minimize the extent to which polymeric material fills the cell windows at the oil/water interfaces formed by the water phase droplets during polymerization. Thus, the presence of electrolyte and the resulting ionic strength of the water phase is believed to determine whether and to what degree the resulting preferred HIPE foams can be open-celled.

Any electrolyte capable of imparting ionic strength to the water phase can be used. Preferred electrolytes are mono-, di-, or trivalent inorganic salts such as the water-soluble halides, e.g., chlorides, nitrates and sulfates of alkali metals and alkaline earth metals. Examples include sodium chloride, calcium chloride, sodium sulfate and magnesium sulfate. For HIPEs that are used to make polymeric foams, calcium chloride has been found to be suitable for use. Generally the electrolyte will be utilized in the water phase of the HIPE in a concentration in the range of from 0.2% to 30% by weight of the water phase. More preferably, the electrolyte will comprise from 1 % to 20% by weight of the water phase.

For HIPEs used to make polymeric foams for ear plugs, a polymerization initiator is typically included in the HIPE. Such an initiator component can be added to the water phase of the HIPE and can be any conventional water-soluble free radical initiator. These include peroxygen compounds such as sodium, potassium and ammonium persulfates, hydrogen peroxide, sodium peracetate, sodium percarbonate and the like. Conventional redox initiator systems can also be used. Such systems are formed by combining the foregoing peroxygen compounds with reducing agents such as sodium bisulfite, L-ascorbic acid or ferrous salts. The initiator can be present at up to 20 mole percent based on the total moles of polymerizable monomers in the oil phase. Preferably, the initiator is present in an amount of from 0.001 to 10 mole percent based on the total moles of polymerizable monomers in the oil phase.

To effect preparation of the polymeric foams preferred for the ear plugs herein, the oil and water phases as hereinbefore described are combined under agitation to form a HIPE emulsion in the form of a stable foam. The weight ratio of the water phase to the oil phase in such a combination will generally range from 10:1 to 100:1, more preferably from 10:1 to 50:1. This HIPE emulsion foam formed from such a combination of oil and water phases is then subjected to polymerization conditions which are sufficient and suitable to bring about polymerization of the monomers in the oil phase and to thereby form a solid cellular foam structure.

The HIPE emulsion, formed as described hereinbefore, will generally be placed in a suitable reaction vessel, container or region to be polymerized. In one embodiment herein, the reaction vessel comprises a tub constructed of polyethylene from which the eventually polymerized solid foam material can be easily removed for further processing after polymerization has been carried out to the extent desired. Alternatively, the HIPE emulsion can be poured into molds of the desired shape for the final ear plug product and then cured in such molds.

Polymerization conditions to which the HIPE emulsion will be subjected will vary depending upon the monomeric and other makeup of the oil and water phases of the emulsion and the type and amounts of polymerization initiators utilized. Frequently, however, polymerization conditions will comprise maintenance of the HIPE emulsion at elevated temperatures of from 55° C. to 90° C., more preferably from 60° C. to 66° C., for a time period ranging from 4 to 24 hours, more preferably from 4 to 12 hours.

The solid HIPE foam which is formed upon completion of the hereinbefore described polymerization step will generally be a flexible, open-cell porous structure having its cells filled with the residual water phase material which was used to prepare the HIPE emulsion prior to polymerization. This foam will generally be dewatered prior to, or more preferably after, being cut or otherwise made ready for use as an ear plug device. Dewatering can be brought about by compressing the foam to squeeze out residual water, by subjecting the foam, or the water therein, to elevated temperatures, e.g., to temperatures from 60° C. to 200° C. or to microwave treatment, or by a combination of both compressing and water heating techniques. The dewatering step of HIPE foam processing will generally be carried out until the HIPE foam ready for use is as dry as practical. Frequently such compression dewatered foams will have a water (moisture) content of from 50% to 500%, more preferably from 50% to 200%, by weight on a dry weight basis. Subsequently, heated foams can be dried to a moisture content of from 5% to 40%, more preferably from 5% to 15%, on a dry weight basis.

After dewatering, it may be useful to wash the HIPE polymeric foam with a C₁₋₄ lower alkanol solvent. Washing the foam with, for example, ethanol or isopropanol can serve to restore and preserve any foam elasticity and resiliency which may have been lost through compression dewatering. Solvent washing may also help remove any residual emulsifier or excess reactants from the foam.

Polymeric foams, including the preferred foams prepared from the HIPE water-in-oil emulsions herein, may be relatively closed-celled or relatively open-celled in character, depending upon whether and/or the extent to which, the cell walls or boundaries, i.e., the cell windows, are filled or taken up with polymeric material. The polymeric foam materials useful in the ear plug structures of the present invention are those which are relatively open-celled in that the individual cells of the foam are for the most part not completely isolated from each other by polymeric material of the cell walls. Thus the cells in such substantially open-celled foam structures have intercellular openings or "windows" which are large enough to permit ready gas or liquid, i.e., fluid, transfer from one cell to the other within the foam structure.

In substantially open-celled structures of the type useful herein, the foam will generally have a reticulated character with the individual cells being defined by a plurality of mutually connected, three dimensionally branched webs. The stands of polymeric material which make up the branched webs of the open-cell foam structure can be referred to as "struts". For purposes of the present invention, a foam material is "open-celled" if at least 80% of the cells in the foam structure are in fluid communication with at least one adjacent cell.

One essential feature of the open-celled foams useful on ear plugs in accordance with the present invention is the size of the cells in the open-celled foam. Foam cells, and especially cells which are formed by polymerizing a monomer-containing oil phase that surrounds relatively monomer-free water-phase bubbles, will frequently be substantially spherical in shape. The size or "diameter" of such substantially spherical cells is thus yet another commonly utilized parameter for characterizing foams in general as well as for characterizing certain preferred ear plug foams of the type utilized in the present invention. Since cells in a given sample of polymeric foam will not necessarily be of approximately the same size, an average cell size, i.e., average cell diameter, will often be specified.

Cell size is a foam parameter which can also impact on a number of important mechanical and performance features of the ear plug foam material of this invention. Cell size, for example, can affect mechanical properties of the foams herein including such features as flexibility and resistance to and recovery from compression deflection.

A number of techniques are available for determining average cell size in foams. These techniques include mercury porosimetry methods which are well known in the art. The most useful technique, however, for determining cell size in foams involves simple photographic measurement of a foam sample. In such a technique, a photomicrograph of a fracture surface of a foam is made. Superimposed on the photomicrograph is a scale representing a dimension of 10 microns. Such a scale can be used to determine average cell size via an image analysis procedure. Image analysis of photomicrographs of foam samples is, in fact, a commonly employed analytical tool which can be used to determine average cell size of the foam structures herein. Such a technique is described in greater detail in Edwards et al; U.S. Patent No. 4,788,225; Issued November 29, 1988.

As determined by direct photographic measurement, the foams useful as ear plugs in accordance with the present invention will preferably have an average cell size ranging from 10 to 250 microns. More preferably, average cell size will range from 20 to 150 microns. Most preferably, average cell size will be between 30 and 100 microns.

Size or diameter of the cells in the foam absorbents herein can be influenced and controlled by variation of the foam composition and processing features. For the preferred HIPE-based foams, these include primarily those factors which determine the size of the water phase "bubbles" in the HIPE emulsion precursor of the polymeric foam formed therefrom. Thus cell size can be varied by adjusting the water to oil ratio of the HIPE emulsion and by adjusting the type and amount of emulsifier used to form the HIPE emulsion. Cell size can also be varied by adjusting the amount of shear agitation, e.g., stirring, by which the HIPE emulsion is formed.

The open-celled foam materials, and preferably the HIPE foam materials, hereinbefore described are formed into a three-dimensional mass of material which can suitably be used for and as ear plugs. Such a mass of foam material must have certain size, flexibility and resiliency characteristics.

Generally the mass of material suitable for use as ear plugs will comprise 0.02 to 1.0 gram of foam material, more preferably from 0.09 to 0.5 gram and most preferably from 0.15 to 0.25 gram of foam material. In its uncompressed state, the mass of foam material suitable for use as ear plugs will typically have a volume of from 1.5 to 4.0 cm³, more typically from 2.0 to 3.5 cm³.

The three-dimensional mass of open-celled foam material useful as an ear plug will also have dimensions in its compressed state which permit the mass of foam material in its compressed state to be inserted into the ear canal of the wearer of the ear plug. Typically, this means that the mass of foam material will be compressible in its cross-section to dimensions which fall within the range of from 2 to 10 mm, more preferably from 3 to 6 mm, without becoming substantially shortened in length. For purposes of this characterization, the "length" of the three-dimensional mass is its major dimension, and its cross-section is taken as the plane which is perpendicular to the axis running along its length.

The mass of open-celled foam material must also have sufficient resistance to compression such that it forms an appropriately tight fit against the walls of the ear canal once the ear plug has been inserted into the ear canal of the wearer. Such resistance to compression of the mass of foam material can be quantified by specifying the static stress exhibited by the foam mass when it is compressed to 50% of its relaxed volume. The mass of foam useful as an ear plug herein is one which exhibits a static stress of from 1 to 15 kPa at 50% compression. More preferably, the foam mass will exhibit a static stress of 5 to 10 kPa at 50% compression. Measurements of static stress on the foam mass may be made by using a Dynamic Mechanical Analysis (DMA) device. Masses of foam material which exhibit these static stress characteristics have been found to exert a pressure of less than 9 kPa on the ear canals of a majority of the population when inserted.

The mass of foam material must also be sufficiently resilient so that it recovers from compression with enough speed and force to form a suitable seal with the ear canal after it has been inserted. Thus a foam mass suitable for use herein is one which is sufficiently resilient to return to at least 90% of its relaxed volume within from 2 to 60 seconds after release from compression to at least 80% of its relaxed of volume.

The mass of material formed from the foam materials described herein may be of any suitable shape or configuration. Thus, prior to insertion into the ear canal, the foam mass may be cylindrical, spherical, conical, frusto-conical, funnel-shaped, parallelpiped-shaped, combinations of these shapes or any irregular variations of these shapes. Foam material may be cut into such shapes from larger masses of foam or foam may be cured in molds to provide masses of foam having the desired shapes without cutting.

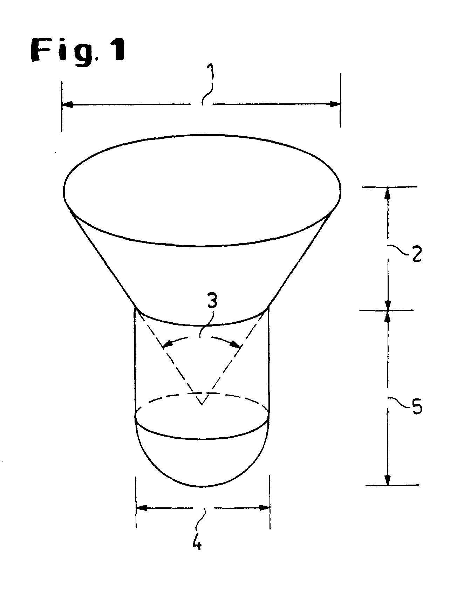

A preferred shape for a molded foam ear plug product is a funnel-shaped piece of foam as shown in Figure 1. A "funnel-shaped" ear plug is one which has a frusto-conical portion of the plug at one of its ends and a cylindrical portion at its other end. In the funnel-shaped Figure 1 configuration, the frusto-conical end has a largest diameter **1** which can range from 10 to 25 mm, with the length **2** of the frusto-conical end ranging from 5 to 15 mm. The angle **3** of the cone can range from 30° to 70°. The cylindrical end of the Figure 1 device has a diameter **4** which can range from 5 to 15 mm and a length **5** which can range from 5 to 10 mm. The cylindrical end of the Figure 1 structure is rounded. The total length **2** + **5** of the funnel-shaped structure of Figure 1 can range from 10 to 25 mm.

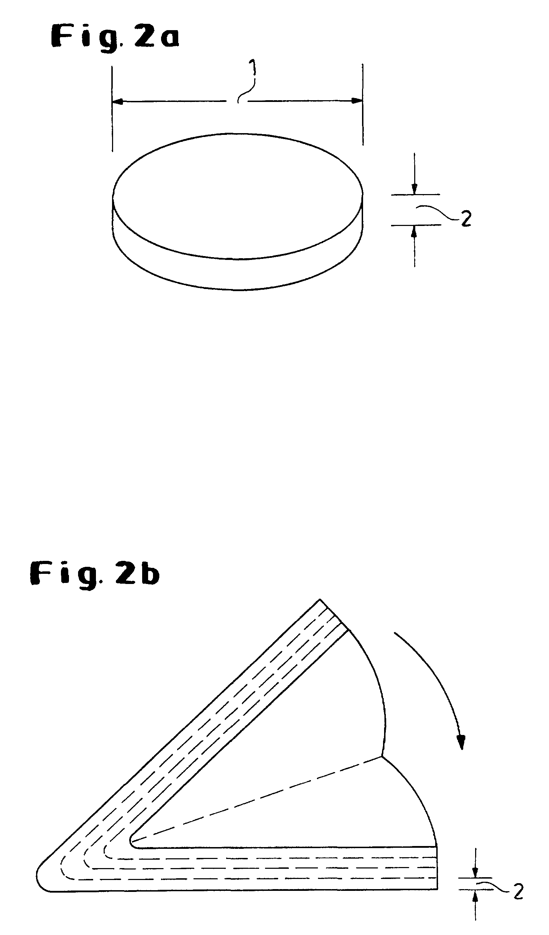

The mass of foam material useful as an ear plug herein may also be formed by folding, rolling or combining pieces of foam which in their unfolded, unrolled or uncombined state would not be of size or shape suitable for insertion into the ear canal. Thus, for example, the foam material can be provided in the form of a relatively flat disc of material which can be repeatedly folded in half, or folded in half and then rolled, to form a conical mass of foam useful as the ear plug. A preferred foam mass of this type, and its folding into an ear plug device, are illustrated in Figure 2a/2b. Alternatively, a piece of relatively flat (1 to 3 mm) foam of any shape can be folded, rolled or combined with similar foam pieces, to thereby provide a foam mass of the type useful herein as ear plugs.

Figure 2a shows a foam disc which can range in diameter **1** from 30 to 60 mm and in thickness **2** from 1 to 3 mm. Figure 2b shows the Figure 2a disc being folded in half for the third time, thereby leading to a generally conical ear plug device having separate layers of foam material of thickness **2** throughout its structure.

Preparation of a suitable foam ear plug is illustrated by the following example.

### EXAMPLE

This example illustrates the preparation of a molded tridimensional HIPE foam suitable for use as an ear plug. An aqueous phase is prepared containing the ingredients shown in Table 1. The oil phase is prepared using the ingredients shown in Table 2.

**Table 1.**

| Aqueous Phase Composition for HIPE. | |
|---|---|
| Component | Foam A |
| Water | QS |
| Potassium Persulfate | 0.05% |
| Calcium Chloride | 4.0% |

**Table 2.**

| "Oil Phase" Composition for HIPE. | |
|---|---|
| Component | Foam A |
| 2-ethylhexyl acrylate | 57.5% |
| styrene | 30% |
| divinyl benzene* | 12.5% |
| Isostearyl diglycerol & hexadecyl diglycerol ether emulsifier | 4%** |

| | |
|---|---|
| * Divinyl benzene in this table is a special blend comprising 45% ethyl styrene and 55% divinyl benzene, unless otherwise specified. | |
| ** Addition level of emulsifier and other adjuvants to the oil phase are "add-on" percentages; monomer composition sums to 100%. The 4% of emulsifier is actually 4 parts per 104 parts. | |

Controlled ratios of the oil phase stream (25°C) and water phase are fed to a dynamic mixing apparatus, described in more detail in US Patent 5,387,207 (Dyer et al.) issued February 7, 1995. Appropriate mixing of the combined streams in the dynamic mixing apparatus is achieved by means of pin impellers in mixing cylinders. The HIPE is produced at a rate of 2.3 kg./min.

Molds in the form of an ear plug similar to the one shown in Figure 1 are then filled with the as-described-above HIPE. The HIPE emulsion is cured therein at a temperature of 65°C for about 18 hours to form molded HIPE ear plug (Foam A). The molded HIPE foam is then stripped from the molds.

The stripped, molded foam articles are then dewatered and according to the following method:
1) Place 2 or 3 of the molded implements onto the apertured surface of a Buchner funnel (~28 cm diameter) that is attached to a 2 liter filtering flask. Place a latex rubber sheet over the top of the Buchner funnel and attach the filtering flask to a vacuum source. Maintain suction until the flow of liquid from the samples is substantially stopped.
2) Remove the samples and place them in hot tap water for 1 minute. Place them back on the Buchner funnel and again cover them with the latex rubber sheet. Attach the filtering flask to a vacuum source and maintain suction until the flow of liquid from the samples is substantially stopped.
3) Repeat Step 2 to provide two washings.
4) Allow the samples to air dry.
5) Repeat Steps 1 to 4 until all the samples have been dewatered.

Table 3 summarizes the conditions under which each HIPE stream was made along with relevant properties of the foam produced from these HIPE streams following curing.

**Table 3.**

| Preparative Conditions and Properties of Foam A. | |
|---|---|
| Property | Foam A |
| Water:Oil Ratio | 16 |
| Mixer RPM | 300 |
| Pour Temperature | 65°C |
| | |
| Av. Cell Size | 60µ |
| Static Stress | 7 kPa |
| Resilient | Yes |
| | |

The plug of funnel-shape prepared in Example 1 is useful, upon insertion into the ear canal of a wearer, as an effective sound/noise attenuator and is comfortable for wearing though the night.

## Claims

1. An ear plug suitable for effectively attenuating loud or bothersome noise and sounds when positioned within the ear canal of a wearer, which ear plug comprises from 0.02 gram to 1.0 gram, preferably 0.09 gram to 0.5 gram, of a three-dimensional mass of an open-celled, compressible foam material having cells which range in size from 10 to 250 microns, preferably from 30 to 100 microns, which mass of foam material:
(i) has dimensions in its compressed state which makes said mass suitable for insertion into the ear canal of the ear plug wearer;
(ii) has a resistance to compression such that said mass exerts a static stress of from 1 to 15 kPa, preferably from 5 to 10 kPa, when compressed to 50% of its relaxed volume ; and
(iii) is sufficiently resilient so as to return to at least 90% of its original relaxed volume within from 2 to 60 seconds after release from compression to at least 80% of its relaxed volume.

2. An ear plug according to Claim 1 wherein said compressible foam material is a polymeric foam prepared by polymerizing a High Internal Phase Emulsion.

3. An ear plug according to Claim 2 wherein said High Internal Phase Emulsion has an oil phase comprising a polymerizable monomer, a polymerizable co-monomer, a cross-linking agent and an emusifier; a water phase comprising an electrolyte; and a water phase to oil phase ratio of from 10:1 to 100:1.

4. An ear plug according to any of Claims 1 to 3 wherein the mass of foam material has a volume in its relaxed state of from 1.5 to 4.0 cm³.

5. An ear plug according to any of Claims 1 to 4 wherein the mass of foam material has cross-sectional dimensions which, in the compressed state of the foam mass, fall within the range of from 2 to 10 mm.

6. An ear plug in accordance with any of Claims 1 to 5 wherein said mass of foam material is formed by folding, rolling or combining one or more flat pieces of foam having a thickness of from 1 to 3 mm.

7. An ear plug in accordance with any of Claims 1 to 6 which is of conical configuration and which has been formed into such configuration by folding in half of a disc-shaped mass of foam having, in its uncompressed state a diameter ranging from 30 to 60 mm and a thickness ranging from 1 to 3 mm, followed by repeated folding in half or rolling to form a foam mass of generally conical configuration.

8. An ear plug in accordance with any of Claims 1 to 5 which is of generally funnel-shaped configuration having, in its uncompressed state, a diameter at its cylindrical end of from 5 to 15 mm, a major diameter at its frusto-conical end of from 10 to 25 mm, and an axial length of from 10 to 25 mm.

9. An ear plug suitable for effectively attenuating loud or bothersome noise and sounds when positioned within the ear canal of a wearer, which ear plug comprises from 0.02 gram to 1.0 gram of a three-dimensional mass of an open-celled, compressible polymeric foam material which has cells which range in average size from 10 to 100 microns and which is prepared by polymerizing a High Internal Phase Emulsion, wherein:
a) the water phase to oil phase ratio in the High Internal Phase Emulsion ranges from 10:1 to 70:1;
b) a combination of monomer, co-monomer and crosslinker in the oil phase in the High Internal Phase Emulsion comprises from 47% to 70% of ethyl hexyl acrylate, from 22% to 40% styrene, and from 8% to 20% di-vinyl benzene; and
c) the mass of foam material has cross-sectional dimensions in its compressed state which fall within the range of from 3 to 6 mm.

10. An ear plug in accordance with Claim 9 which is of generally funnel-shaped configuration having, in its uncompressed state, a diameter at its cylindrical end of from 5 to 15 mm, a major diameter at its frusto-conical end of from 10 to 25 mm, and an axial length of from 10 to 25 mm.

11. An ear plug in accordance with Claim 9 which is of conical configuration and which has been formed into such configuration by repeated folding in half of a disc-shaped mass of foam having, in its uncompressed state a diameter ranging from 30 to 60 mm and a thickness ranging from about 1 to 3 mm.
